(19) Europäisches Patentamt
European Patent Office
Office européen des brevets

(11) **EP 4 376 014 A1**

(12) **EUROPEAN PATENT APPLICATION**

(43) Date of publication:
**29.05.2024 Bulletin 2024/22**

(21) Application number: **23159107.4**

(22) Date of filing: **28.02.2023**

(51) International Patent Classification (IPC):
**G16H 10/60** (2018.01) **G06F 16/20** (2019.01)
**G06F 17/00** (2019.01)

(52) Cooperative Patent Classification (CPC):
**G16H 10/60; G06F 16/2228; G06F 16/243;
G06F 16/9024; G06N 3/0455; G06N 3/0475;
G06N 3/084; G06N 3/094**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.11.2022 US 202263384798 P**

(71) Applicant: **Siemens Healthineers AG
91301 Forchheim (DE)**

(72) Inventors:
• **Boettger, Thomas
91054 Erlangen (DE)**
• **Ullaskrishnan, Poikavila
Lebanon, 03766 (US)**
• **Varadan, Yamini
East Brunswick, 08816 (US)**
• **Yu, Daphne
Yardley, 19067 (US)**

(74) Representative: **Siemens Healthineers
Patent Attorneys
Siemens Healthcare GmbH
SHS TE IP
Henkestraße 127
91052 Erlangen (DE)**

(54) **METHOD AND SYSTEM FOR PROVIDING INFORMATION FROM AN ELECTRONIC MEDICAL RECORD DATABASE**

(57) Provided are methods and systems for the targeted retrieval of information from an electronic database based on or as a response to a query comprising natural language and provided by a human user. Specifically, a computer-implemented method for providing information (I) from an electronic medical record database (EMR) is provided. The method comprising the following steps of receiving (S10) a query (Q) for the information (I), identifying (S20) at least one code (IC) of a medical ontology (MO) on the basis of the query (Q), determining (S30) one or more related codes (RC) in the medical ontology (MO) based on the medical ontology (MO) and the identified at least one code (IC), the one or more related codes (RC) having a relation to the at least one identified code (IC) in the medical ontology (MO), parsing (S40) the electronic medical record database (EMR) so as to identify entries (EN) in the electronic medical record database (EMR) comprising one or more of the identified at least one code (IC) and the one or more related codes (RC), and providing (S50) the information (I) on the basis of the identified entries (EN).

FIG 3

**Description**

The invention concerns the field of data retrieval based on a query. In particular, the invention concerns the targeted retrieval of information from an electronic medical record database based on or as a response to a query comprising natural language and provided by a human user.

Hospitals produce enormous amounts of data per year. This mass of information comprises clinical notes, lab tests, medical images, sensor readings, genomics, operational, and financial data. At the same time, much of this information is unused. Relevant information is lost between the cracks of different healthcare departments, e.g., from radiology to therapy, leading to multiple re-diagnoses, loss of time and loss of opportunities for faster and more precise therapy.

Although vast amounts of data are in principle available, leveraging this information in the clinical routine is very difficult. For coming to a clinical decision, a number of relevant questions need to be answered by the treating physician. These questions may pertain to the clinical history of the patient under consideration (e.g.: Was computed tomography angiography done?) or the decisions taken for comparable cases (e.g.: Are there patients with comparable disease states on record?). Properly answering these questions requires the treating physician to manually search in the electronic health record or in multiple picture archiving and communications systems and, in the worst case, re-examine prior studies such as radiology images again.

What is more, the data on record, such as prior medical reports for the patient or diagnosis for similar cases, have typically not been generated by the treating physician herself but by colleagues from different disciplines such as radiologists, pathologists and so forth. In addition, the existing data entries typically are not focused on the information currently sought for. This makes it very difficult for the physician to directly identify the information she or he is looking for with the consequence that a physician would have to go through all of the documents she or he identified as potentially relevant one by one. One major drawback is that this is inherently error prone. Another, maybe even more severe drawback lies in the fact that the manual lookup consumes enormous amounts of time.

The lack of an efficient search infrastructure (which, in turn, would need efficient data integration, indexing and query capabilities) is the main reason for this massive healthcare problem. Despite of the fact that this is an ubiquitous problem which has been targeted multiple times, a coherent solution is still at large. Solutions hitherto proposed often never reached satisfiable adoption rates due to lack of standardization and high costs of migration and change without the promise of interoperability. The complexity of healthcare with millions of concepts and relations at multiple granularity levels makes the search problem very difficult to tackle - especially when the solutions are required to meet the requirements of the healthcare IT industry in terms of performance, data and process security, interoperability, as well as cost efficiency.

It is therefore an object of the invention to provide methods and systems allowing for an efficient provision of information from an electronic medical record database based on a query containing natural language. In particular, it is an object to provide methods and systems that allow for a comprehensive retrieval of the relevant data items and that, at the same time, readily adapt to updated data standards and changing medical concepts while ensuring low maintenance and development efforts.

This object is, in particular, solved by a method for providing information from an electronic medical record database, a corresponding system, a corresponding computer-program product, and a computer-readable storage medium according to the independent claims and aspects as herein described. Alternative and/or preferred embodiments are object of the dependent claims and the further aspects and examples as herein described.

In the following, the technical solution according to the present invention is described with respect to the claimed systems as well as with respect to the claimed methods. Features, advantages, or alternative embodiments described herein can likewise be assigned to other claimed objects and vice versa. In other words, claims addressing the inventive method can be improved by features described or claimed with respect to the systems. In this case, e.g., functional features of the methods are embodied by objective units or elements of the systems.

The technical solution will be described both with regard to methods and systems for providing information and also with regard to methods and systems for providing trained functions. Features and alternate forms of embodiments of data structures and/or functions for methods and systems for providing can be transferred to analogous data structures and/or functions for methods and systems for providing trained functions. Analogous data structures can, in particular, be identified by using the prefix "training". Furthermore, the trained functions used in methods and system for providing information can, in particular, have been adjusted and/or trained and/or provided by methods and systems for adjustment of trained functions.

According to an aspect, a computer-implemented method for providing information from an electronic medical record database is provided. The method comprises a plurality of steps. One step is directed to receiving a query for the information. Another step is directed to identifying at least one code of a predetermined medical ontology in the query. Another step is directed to determining one or more related codes in the medical ontology based on the medical ontology and the identified at least one code. Another step is directed to parsing the electronic medical record database so as to identify entries in the electronic medical record database indicating (or comprising or referencing) one or more of the identified at least one code and the one or more related codes. Another step is directed to providing the information on the basis of the identified entries.

According to some examples, the medical record database may comprise one or more data storages. In particular, the medical record database may comprise one or more local or distributed data storages. Alternatively or additionally, the medical record database may comprise one or more cloud storage modules. According to some examples, the medical record database may comprise or may be embodied as a Picture Archiving or Communication System (PACS). Further, according to some examples, the medical record database may comprise or may be embodied as an Electronic Heath Record (EHR). Further, according to some examples, the medical record database may comprise or may be embodied as Radiology Information System (RIS) or Laboratory Information System (LIS). According to some examples, the medical record database may be part of a healthcare information system operating in a given healthcare organizations such a hospital or hospital chain.

According to some examples, the medical record database may be configured to support one or more predetermined data standards. In other words, the medical record databases may be formatted according to the one or more predetermined data standards. This may mean that the data entries in the medical record database are formatted according to the one or more predetermined data standards.

According to some examples, the predetermined standard comprises a plurality of predefined data fields which have a predetermined data identifier with which the content of the data fields can be called (based on which the content of the data fields can be accessed). As such, the data fields may be seen as placeholders for specific data contents. For instance, these contents may be image data, text data, numeric data and any combination thereof. According to some examples, the one or more predetermined data standards comprise the Digital Imaging and Communications in Medicine (DICOM) standard and/or the Fast Healthcare Interoperability Resources (FHIR) standard.

Entry as used herein may refer to individual data entries in the medical record database. Typically, each entry can be attributed to at least one patient. According to some examples, each entry may be attributed to precisely one patient. According to some examples each entry may comprise patient data of a patient. An entry may comprise medical image data and/or non-image data. In particular, an entry may comprise a medical report indicating on or more findings of the patient.

The medical image data may be a two- or three-dimensional radiology image data set depicting a body part of a patient and having been acquired using a medical imaging modality such as a computed tomography system (CT system), a magnetic resonance system (MR system), an angiography (or C-arm X-ray) system, a positron-emission tomography system (PET system) or the like. Further, the medical image data set may be a two-dimensional pathology image data set, i.e., a so-called whole-slide image.

The non-image data may comprise numeric data and/or text data. For instance, the non-image data may comprise a structured or unstructured text document indicating one or more findings of the patient. In addition to that or as an alternative, the non-image data may comprise lab data, vital values, or supplementary information such as demographic information of a patient.

According to some examples, the entries may comprise indications of one or more medical findings which may have been generated by computer-aided diagnosis tools or by a diagnosing physician. According to some examples, the entries may be pushed to the medical record database by the computer-aided diagnosis tool and/or a data generating instrument such as an imaging modality and/or a human user. Upon pushing an entry to the medical record database, the entry may be formatted according to a predetermined data standard.

According to some examples, the query may be input by a user such as physician. According to some examples, the query may comprise natural language. According to some examples, receiving the query may comprise obtaining an utterance from a user, e.g., via a user interface comprising a microphone. According to some examples, receiving the query may comprise obtaining a text input by a user, e.g., via a keyboard of a user interface.

According to some examples, the query may comprise a question of the user relating to entries of the medical record database. According to some examples, the query may relate to a distinct patient such as the patient currently under consideration. According to some examples, the query may relate to a plurality of patients. For instance, the query may be of the following kinds: "show all scans of the patient imaging the liver", "show all patients with lesions in the left coronary artery structure", or "show similar patients".

According to other examples, the query may be a data query automatically generated, e.g., by another method or system. Suchlike query may likewise comprise natural language. As an alternative or in addition to that, suchlike query may also comprise electronic data identifiers such as accession numbers or other data IDs.

In particular, a (medical) ontology is a formally ordered representation of a set of elements and the relationships between them. In particular, the elements of a medical ontology may relate to medical concepts, medical data and/or medical information. An ontology can be used in particular to exchange information in digitized and formal form between application programs and services. In particular, a medical ontology represents a network of concepts, data and/or information with logical relations. An ontology can, in particular, contain inference and integrity rules, i.e., rules for conclusions and for ensuring their validity. Further according to some examples, the ontology may comprise a hierarchy of elements where lower-level elements are linked to higherlevel elements. In particular, an ontology may comprise a number of hierarchical levels.

A (medical) ontology can be represented, in particular, in the form of a mathematical graph comprising nodes and edges, in particular in the form of a directed graph. In this case, the nodes and/or the edges can, in particular, have further data.

A medical ontology may, in particular, be an ontology that relates to medical and/or (human) biological issues. A medical ontology is, in particular, independent of the specific patient, in other words, a medical ontology represents and structures existing abstract technical or domain knowledge. Such a medical ontology can be the result of scientific research, in particular with regard to the causal relationships and structure of medical information. In particular, the medical ontology is independent of the medical record database and the entries therein comprised.

According to some examples, the medical ontology is predefined. In particular, the medical ontology may be externally provided. According to some examples, the medical ontology may be public.

A code may relate to an element (concept, data item and/or information) of the medical ontology. In particular, each code may be configured to unambiguously identify an element (concept, data item or information) of the medical ontology. In particular, each code may be configured to unambiguously identify a node (or edge) of the medical ontology.

According to some examples, the codes may be predefined based on the medical ontology. In other words, a set of predefined codes is provided based on the medical ontology, wherein the step of identifying the at least one code and the step of determining one or more related codes respectively comprises a selection from the set of predefined codes. "Code of the medical ontology" may mean a code of the set of predefined codes.

According to some examples, the codes may be identical to the nodes or part of the nodes of the medical ontology.

According to some examples, the step of identifying the at least one code in the query may comprise identifying a notion relating to a distinct code of the medical ontology in the query and identifying the distinct code as the at least one code. According to some examples, the step of parsing may comprise identifying entries comprising notions relating to the at least one identified code and/or the one or more related codes.

According to some examples, related codes are codes having a relation to the at least one identified code in the medical ontology. The relation may be predefined or predetermined or dynamically determined. According to some examples, the relation may be a spatial relation within the medical ontology and/or a logical relation within the medical ontology. According to some examples, related codes are codes within a predetermined distance in the medical ontology from an identified code and/or related codes are codes in the medical ontology having a predetermined similarity to an identified code. According to some examples, related codes are codes which are neighboring an identified code in the medical ontology. According to some examples, related codes are codes which are directly connected to an identified code in the medical ontology (i.e., which are connected via a shared edge in the medical ontology without any further code in between).

"Information" relates to the output of the method that is provided, e.g., to a user or to another method or system. The information is based on the entries retrieved from the medical record database based on the identified codes and the determined related codes. "Based on the entries" may mean post-processing the entries so as to generate the information. According to some examples, the information may comprise excerpts from the entries, such as images or data values.

According to some examples, the information may comprise a visual representation configured to be displayed to a user in a user interface. In particular, the representation may comprise text, images, and/or values in a human interpretable format.

It is an idea of the invention to use codes of a medical ontology as search terms to query a medical record database. Further, by determining related codes based on the medical ontology, the search space can be effectively broadened without loosing specificity.

One major advantage of this approach is that by using existing and even public knowledge in the form of the ontology, a reliable and easy to implement way to query data systems for information can be provided. Importantly, it is neither required to structure the database itself according to the ontology nor to define or at least learn relations between query inputs and information outputs for each application/field of operation. At the same time, the solution is very easy to maintain. Individual components like the database structure or the ontology can be easily changed independently from one another. Even if the ontology is updated, for instance, the step of determining related codes is still applicable to broaden the search space. Comparted to other approaches, it will not be required to reconfigure learnt relations between expressions.

In addition, since the method requires only small sets of codes to be mapped to the input data and since it uses existing ontologies, the development and initial deployment speeds are higher.

In addition, the method is able to cope very well with unseen queries. Typically, dedicated query engines are designed to provide results only if exact answers are available. By contrast, the methods and systems proposed here, due to the extension of queried codes using the ontology, are able to return results even if none matches the query to 100%. At the same time, this renders the method more user friendly, as end-users cannot be expected to ask the question in the most appropriate manner. Also in such cases, the degree of flexibility introduced by basing the search on the ontology will help the user find the required data easier.

According to an aspect, the medical ontology is based on SNOMED.

SNOMED (or SNOMED CT or SNOMED Clinical Terms) is a systematically organized computer-processable collection of medical terms providing codes, terms, synonyms and definitions used in clinical documentation and reporting. SNOMED is considered to be the most comprehensive, multilingual clinical healthcare terminology in the world. Accordingly, the usage of SNOMED for the medical ontology facilitates an efficient and at the same time comprehensive data retrieval. For further information, reference is made to https://www.snomed.org/.

According to an aspect, the medical ontology is based on RadLex.

RadLex is a terminology with a focus on radiology the development of which is coordinated by the RSNA. Being specifically designed for radiology applications, the usage of RadLex facilitates an efficient and at the same time comprehensive data retrieval especially in PACS systems.

According to an aspect, the method further comprises obtaining the medical ontology (e.g., from a medical ontology database) .

According to an aspect, the step of obtaining the medical ontology comprises selecting the medical ontology from a plurality of different candidate medical ontologies based on the query and/or supplementary information.

According to some examples, the supplementary information may be clinical information related to the query. According to some examples, the clinical information may, for instance, relate to or comprise a diagnostic task, the user has to perform (when formulating the query), a stage of a patient in her or his clinical trajectory, an organ concerned, and/or a suspected disease or the like.

Specifically selecting the medical ontology has the advantage that a more targeted retrieval of related codes can be performed. In turn, the parsing step is rendered more accurate.

According to an aspect, the method further comprises obtaining a set of predefined codes based on the medical ontology as the codes of the medical ontology. Thereby, the step of identifying the at least one code comprises selecting the at least one code from the set of predefined codes and the step of determining the one or more related codes comprises selecting the one or more related codes from the set of predefined codes.

The usage of predefined codes based on the medical ontology has the advantage that the search space can be easily defined and that logical relations within the ontology can be taken automatically into account.

According to an example, the ontology comprises a graph data structure comprising a plurality of nodes, wherein each code corresponds (or unambiguously identifies) one of the plurality of nodes. With that, an efficient search can be facilitated as relations among nodes can automatically be taken into account.

According to an aspect, the method comprises determining if the query is directed to retrieve information for a distinct patient, wherein, in the step of parsing only entries relating to the distinct patient are identified.

With that, the search is rendered more efficient and the user is provided with more specific information.

Such a directed query may for instance occur, if the user would like to see further information for the patient case she or he is currently reviewing. The fact that the query is directed to a distinct case may show in the query itself, e.g., if a concrete patient is mentioned in the query. Alternatively, this may be evident from the circumstances of the query. Accordingly, the step of determining if the query is directed to retrieve information for a distinct patient may be based on the query. Alternatively or additionally, the step of determining if the query is directed to retrieve information for a distinct patient may comprise obtaining circumstances of the query and determining if the query is directed to retrieve information for a distinct patient based on the circumstances. Such circumstances may comprise the diagnostic task, the information already retrieved from the medical record database and currently looked at by the user etc.

In the same way, the method may determine if the query is directed to retrieve information for a plurality of different patients and, in the step of parsing, identify entries relating to the plurality of different patients.

According to an aspect, the medical record database is formatted according to a predetermined data standard.

According to some examples, the predetermined data standard is selected from the Fast Healthcare Interoperability Resources (FHIR) standard, the Digital Imaging and Communications in Medicine (DICOM) standard and any combination of the aforesaid. According to some examples, the predetermined data standard is independent from the medical ontology.

The usage of a predetermined data standard has the advantage that the method integrates well with the existing infrastructure. In addition, this ensures the scalability and maintenance of the solution. In particular, the FHIR standard has gained substantial traction within health IT industries and, thus, enables an efficient search and ensures interoperability. The same holds true for the DICOM standard which is the prevailing standard for radiology applications.

According to some examples, the method further comprises identifying a patient under consideration based on the query and/or the identified clinical context (as herein described), wherein in the step of parsing only entries pertaining (or being associated) to the patient under consideration are considered.

In particular, the clinical context may pertain to the patient the user is currently diagnosing - which, for instance, may become evident from the data already opened in a viewer or the like.

With that, the patient may be automatically detected and the search may be correspondingly limited. Accordingly, the search is more focused which lowers the computational effort and makes the search results are more readily useable.

In other words, the above aspect provides, a computer-implemented method for providing information from an electronic medical record database. The method comprises a plurality of steps. One step is directed to receiving a query for the information. Another step is directed to identifying at least one search term in the query. Another step is directed to determining one or more related search terms based on a medical ontology the related search terms having a relation to the identified at least one search term according to the medical ontology. Another step is directed to parsing the electronic medical record database based on the at least one search term and the related search terms so as to provide the information. Another step is directed to providing the information.

The at least one search term may be equated with the at least one code identified in the query. The one or more related search terms may be equated with the one or more related codes.

According to an aspect, the query comprises natural language (in particular: natural language text) and the method further comprises providing a natural language processing algorithm configured to identify codes of the medical ontology in natural language and inputting the query in the natural language processing algorithm so as to identify the at least one code of the medical ontology in the query.

The natural language algorithm may be configured to recognize and/or understand natural language and, in particular, individual items such as words in input containing natural language. The natural language processing algorithm may be based on a trained or machine-learned function. As an alternative, the natural language processing algorithm may be rule-based. Providing the natural language algorithm may comprise holding the algorithm available in a suitable storge accessible by the computing unit executing the method. Further, providing the algorithm may comprise downloading it by said computing unit.

By using a natural language processing algorithm, in particular, queries from human users may be efficiently processed and reliably mapped to the codes of the medical ontology.

In general, a machine-learned function mimics cognitive functions that humans associate with other human minds. In particular, by training based on training data, the machine-learned function is able to adapt to new circumstances and to detect and extrapolate patterns. Other terms for machine-learned function, may be trained function, trained machine learning model, trained mapping specification, mapping specification with trained parameters, function with trained parameters, algorithm based on artificial intelligence, or machine learned algorithm.

In general, parameters of a machine-learned function can be adapted by means of training. In particular, supervised training, semi-supervised training, unsupervised training, reinforcement learning and/or active learning can be used. Furthermore, representation learning can be used. In particular, the parameters of the machine-learned function can be adapted iteratively by several steps of training. In the presented case, codes of the medical ontology could be attributed to sample queries by an expert. This annotated data can then be used as a ground truth to train the machine-learning function

In particular, a machine-learned function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

For instance, the machine-learned function may be configured to extract one or more items such individual words and their context from the query and map/classify that into the result space of the codes to be identified. This has the advantage that codes can be reliably identified and also the context of the codes can be taken into account. The latter is important if the query indicates that a certain code is to be excluded (for instance: "Show all patients not having had a radiotherapy treatment").

The usage of machine-learned functions in general has the advantage that a more comprehensive and faster screening of the available information can be made. In this regard, machine-learned functions may identify patterns and attributes in the available data that are not accessible for a human.

According to some examples, the natural language processing algorithm comprises a transformer network. According to some examples, the trained function comprises a transformer network.

A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer preferably there is an attention mechanism. The attention mechanism, sometimes denoted as self-attention, is able to relate data items such as words in the query to other data items within the query. The self-attention mechanism for instance allows the model to examine a word within the query and determine the relative importance of other words within the query for the word being examined. Further, the transformer network may comprise a classification module configured to map the output of the encoder or decoder to a set of learned outputs, which are the codes of the medical ontology in the present case.

Training of a transformer model according to some examples may happen in two stages, a pretraining and a finetuning stage. In the pretraining stage, a transformer model may be trained on a large corpus of data to learn the underlying semantics of the problem. Such pretrained transformer models are available for different languages. For certain applications described herein, the fine-tuning may comprise further training the transformer network with medical texts with expert annotated meanings and/or medical ontologies such as RadLex and/or SNOMED.

An advantage of transformer networks is that, due to the attention mechanism, transformer networks can efficiently deal with long-range dependencies in input data. Further, encoders used in transformer networks are capable of processing data in parallel which saves computing resources in inference.

According to an aspect, the query comprises natural language (in particular: natural language text) and the step of identifying comprises identifying the at least one code of the medical ontology based on the natural language.

According to an aspect, the method further comprises determining a linguistic context related to the at least one identified code and determining the related codes and/or parsing the database and/or providing the information is based on the identified linguistic context. For instance, linguistic context may relate to the presence or absence of a medical finding. In particular, the linguistic context may be a negation ("show all patients not having a patellar luxation") or an affirmation of the finding ("did the patient have a CT examination") .

By taking the linguistic context into account, the directed retrieval of the information may be facilitated. In particular, unwanted information may be filtered that would otherwise be retrieved. In particular, determining the linguistic context may be performed by the accordingly configured natural language processing algorithm.

According to an aspect, the step of determining one or more related codes comprises obtaining the medical ontology as a graph data structure, providing a graph search algorithm configured to determine related codes in graph data structures and inputting the medical ontology and the at least one identified code in the graph search algorithm so as to determine the one or more related codes.

A graph data structure can be seen as a data structure encoding the elements of the medical ontology and, therewith the codes of the medical ontology. In particular, the graph data structure may encode the nodes and edges of the medical ontology.

The graph-search algorithm is configured to search graph data structures for related elements. With that, graph-search algorithms are capable of identifying related codes with respect to any code identified in the query.

According to some examples, the graph-search algorithm may comprise a machine-learned function. Alternatively or additionally, the graph search algorithm may implement one or more predefined selection rules. According to another example, related codes may be pre-defined altogether for individual codes of the ontology.

The machine-learned function may be configured as described above. Training such machine-learned functions may involve providing an expert-annotated set of related codes for a reference code. This expert-annotated set of related codes may then be used as a ground truth for training a machine-learned function based on the reference code and the medical ontology.

According to some examples, the graph search algorithm implements a graph traversal-based search scheme or a graph exploration-based search scheme.

According to some examples, the graph traversal-based search scheme comprises a graph hierarchical-recursive search scheme, a lateral recursive search scheme, and/or any combination of the aforesaid schemes.

A hierarchical-recursive search scheme may be configured to search in the hierarchy of the medical ontology for related codes. For instance, for a given code, all codes in the following and preceding hierarchal level of the medical ontology may be identified as related codes. According to some examples, the hierarchical search scheme only identifies codes as related if they belong to the same branch as the code of origin (i.e., the identified code). Recursive may mean that a plurality of hierarchical levels may be subsequently considered.

A transversal-recursive search scheme may be configured to "jump" between neighboring branches for identifying related codes. On the other hand, a transversal-recursive search scheme may focus on the same hierarchical level as the code of origin (i.e., the identified codes). According to some examples, a transversal recursive search scheme may identify only codes of the same hierarchical level as related codes. Recursive may mean that a plurality of neighboring branches may be sequentially considered.

According to some examples, the hierarchical-recursive and the transversal-recursive search schemes may be combined.

According to some examples, the graph traverse-based search scheme comprises a depth-first search scheme configured to first apply a hierarchical recursive search scheme and/or a breadth-first search scheme configured to first apply a lateral-recursive search scheme.

"First" in this respect may mean that the respective search scheme is firstly applied before arbitrary further search schemes may be applied. Thereby, the results of the firstly applied search scheme may be used as starting points for the further search scheme. For instance, each related node determined in a hierarchical search may be a starting point for a transversal search and vice versa.

According to some examples, the graph-exploration-based search scheme implements a greedy search scheme. A greedy algorithm is any algorithm that follows the problem-solving heuristic of making the locally optimal choice of codes in the medical ontology at a given search stage. Despite greedy algorithms conceptually are not efficient in providing the optimal solution, they are very effective in rapidly providing a good approximation which is often sufficient for the underlying problem.

According to some examples, the graph-exploration-based search scheme implements a guided random walk search scheme in the medical ontology. With a random-walk-based search scheme, good convergence to reasonable results can be reached.

According to some examples, the graph-exploration-based search scheme implements a similarity-based search scheme. A similarity based search scheme may be configured to determine a (degree of) similarity between individual codes (elements) of the medical ontology and determine those codes as related which have a sufficient (degree of) similarity to the at least one identified code. According to some examples, the degrees of similarity may dependent on the place of an element/code within the ontology as well as the particular content or concept the element/code stands for in the ontology.

Obtaining (e.g., the medical ontology graph search algorithm) may mean receiving the corresponding item (e.g., from a corresponding database). Further, this may mean holding the corresponding item available in a storage or memory or hosting the corresponding item in a computing unit.

Providing the medical ontology as a graph data structure and, optionally, the usage of graph search algorithms, has the advantage that related codes can be easily and at the same time systematically defined.

According to an aspect, the step of determining one or more related codes comprises obtaining the medical ontology as a graph data structure and determining the one or more related codes based on the graph data structure.

For instance, the related codes may be determined using predetermined selection rules or predetermined related codes. Additionally or as an alternative, predetermined related codes may be (heuristically) defined for each code.

According to an aspect, the step of determining related codes comprises obtaining a search algorithm configured to determine related codes in medical ontologies and inputting the medical ontology and the at least one identified code in the search algorithm so as to determine the one or more related codes.

By using a search algorithm, related codes may be easily and efficiently provided without user interaction.

According to an aspect, the step of obtaining the graph search algorithm comprises providing a plurality of different graph search algorithms and selecting the graph search algorithm from the plurality of different graph search algorithms based on the at least one identified code and/or a type of the medical ontology and/or the query.

According to some examples, the step of selecting comprises determining a clinical information from the identified code and/or the type of the medical ontology and/or the query and selecting the graph search algorithm based on the clinical information.

The clinical information may, for instance, relate to or comprise a diagnostic task, the user has to perform, a stage of a patient, an organ concerned, and/or a suspected disease or the like.

According to some examples, the different graph search algorithms may comprise any one of the graph search algorithms herein described. In particular, the different graph search algorithms may respectively implement one of the search schemes herein described. In particular, the different graph search algorithms may respectively implement a traversal-based search scheme, a exploration-based search scheme, a depth-first search scheme, a breadth-first search scheme, a hierarchical recursive based search scheme, a lateral recursive based search scheme or a greedy search scheme.

According to some examples, the step of selecting is based on minimizing the estimated time for determining the one or more related codes.

In other words, different search strategies may be used for different circumstances of the case. This may allow for a more robust determination of related codes and, thus, overall better search results.

According to an aspect, providing a plurality of graph search algorithms comprises providing at least one first graph search algorithm implementing a depth-first search scheme or a hierarchical recursive search scheme, and at least one second graph search algorithm implementing a breadth-first search scheme or a lateral recursive search scheme.

In other words, with the two groups, two different types of search strategies are provided: one which is focused on exploring the hierarchy of the medical ontology (first) and one which is focused on looking for transversal relations in neighboring branches (first). For different situations different search strategies may be more useful than others. By implementing the different strategies, the broadening of the search space through the related codes can be improved. Thereby, the provision of the above groups complements the structure and logic of medical ontologies.

According to some examples, selecting the graph search algorithm comprises selecting either one of the at least one first graph search algorithms or one of the at least one second graph search algorithms.

According to some examples, providing a plurality of graph search algorithms comprises providing at least one third graph search algorithm implementing a search scheme different than the at least one first graph search algorithm and the at least one second graph search algorithm (in particular: an exploration-based scheme) and selecting the graph search algorithm comprises selecting either one of the at least one first graph search algorithms or one of the at least one second graph search algorithms or one of the at least one third graph search algorithms.

According to some examples, selecting may comprise selecting one of the first graph search algorithms if the clinical information indicates an organ or body part (of a patient).

In other words, the method is focused on a hierarchical search if the information sought for relates to an organ or body part. This is based on the insight that for information on distinct organs/body parts also information on the superordinate organs/body parts as well as sub-organs/body parts are relevant and should be identified in the medical record database.

According to some examples, selecting may comprise selecting one of the second graph search algorithms if the clinical information indicates a disease.

In other words, the method is focused on a transversal search if the information sought for relates to a disease. This is based on the observation that manifestations of a disease are often reflected in different organs but on comparable levels of detail.

According to an aspect, the method further comprises ranking the entries based on the number of references to the at least one identified code and the number of references to the determined one or more related codes respectively comprised in each entry, wherein the step of providing comprises displaying the entries according to the ranking.

According to some examples, the entries are displayed as graphical user interface (GUI) elements. For instance, the GUI elements may be tiles. In particular, the tiles may give a preview of the contents of the entry. Further, the tiles may be configured such that a user may select the tiles (e.g., by clicking on them) for additional content.

In other words, an entry may be ranked higher the more notions of the identified and determined codes it has. This is beneficial since the users attention can be focused on information which is, based on the search, likely more relevant. Thereby, the number of occurrences of the codes is an objective criterion which can be evaluated based on the analysis of the underlying data of the entries.

According to some examples, ranking comprises assigning different weights to the identified at least one code and the determined related codes and ranking the entries additionally based on the assigned weights.

According to some examples, the weights are assigned such that the at least one identified code weighs more in the ranking process than the determined one or more related codes.

By introducing weights, a more differentiated ranking can be effected, e.g., based on the distance an individual code has from the at least one identified code in the query. With that, it can be ensured that a user is pointed to entries with a direct relation to the query with a higher priority.

According to an aspect the step of providing comprises providing a post processing function configured to process the entries, optionally, based on the query or one or more of the identified at least one code and the one or more related codes, so as to generate one or more human-interpretable observables, applying the post-processing function to the data entries, and providing the human-interpretable observables.

According to some examples, the human-interpretable observable comprises a time curve of measurement values and the post processing function is configured to extract a time series of the measurement values from the entries based on one or more of the identified at least one code and the one or more related codes.

According to some examples, the human interpretable observable comprises a key image representing the respective entry and the post-processing function is configured to extract a key image from an entry based on one or more of the identified at least one code and the one or more related codes. A key image may be seen as an image element representing or summarizing contents of an entry in graphical form. Specifically, a key image may be extracted from the image data comprised in an entry and show a particularly meaningful view of a study, optionally with important findings highlighted.

With that, the information is not only provided but also brought into a format that is readily interpretable by a human user. With that, a user is relived from the burden of looking into each of the identified entries one-by-one. Instead, the user is offered already processed information which she or he can readily access. Optionally basing the post processing on the query and/or the codes has the additional advantage that a targeted processing can be ensured so that the observables optimally complement the query. For instance, if the user asked for the progress of a radiotherapy treatment, a corresponding observable may be provided.

According to an aspect, the step of providing comprises providing, optionally based on the query or one or more of the identified at least one code and the one or more related codes, a post processing function configured to classify entries, applying the post-processing function to the data entries so as to generate a classification result per entry, and providing the information based on the classification results.

For instance, a classification result may be whether or not the respective entry fits the query. Specifically, a classification result may be whether or not the entry represents a certain pathology reflected in the query.

By providing a classification function as post-processing function, the entries found upon parsing can be further filtered. With that, the information is rendered more specific further contributing to the efficiency and usability of the method.

According to an example, the step of providing the post processing function comprises determining a clinical context of the query based on the query and/or the at least one identified code and selecting the post-processing function from a plurality of different predetermined post-processing functions based on the determined clinical context.

According to an aspect, the method further comprises generating a synopsis for each entry, optionally based on the query or on one or more of the identified at least one code and the one or more related codes, wherein the step of providing comprises providing the synopsis per entry.

According to some examples, the synopsis may comprise natural language text. According to some examples, the synopsis may comprise a key image derived from the respective entry.

According to some examples, the step of generating a synopsis comprises providing a natural language processing algorithm configured to generate a synopsis of an entry and applying said natural language processing algorithm to the entry. In terms of the natural language processing algorithm, algorithms as described above may be used. According to other examples, the generation of the synopsis may be rule-based, e.g., by auto-filling a predetermined template.

According to some examples, the synopsis may be seen as a human-interpretable observable and the post-processing function is configured to generate a synopsis of an entry based on one or more of the identified at least one code and the on or more related codes. In particular, suchlike post-processing function may comprise a natural language processing function.

With a synopsis, the user is provided with an overview she or he can readily interpret. With that, additional support can be provided for a user. By basing the generation of the synopsis on the code or the query, the synopsis can be rendered more specific.

According to an aspect, at least one of the at least one identified code and the related codes is associated with a numerical value, and the method further comprises determining a threshold for the numerical value based on the query, wherein, in the step of providing, entries are selected based on the threshold and the numerical value of the respective entry, wherein the information is based on the selected entries.

According to some examples, the information is only based on entries the numerical value of which is above the determined threshold.

According to some examples, the numerical value is associated with the at least one identified code.

According to some examples, the threshold is related to the presence or absence of a medical finding.

According to some examples, determining the threshold comprises identifying linguistic context related to the medical finding in the query and setting the threshold based on the identified linguistic context. For instance, linguistic context may relate to the presence or absence of the medical finding. In particular, the linguistic context may be a negation of the finding ("show all patients not having a patellar luxation?") or an affirmation of the finding ("Are there any true bifurcation lesions?").

According to some examples, the linguistic context is identified by applying a natural language processing algorithm to the query. The natural language algorithm may be same as used for identifying the at least one code or a different one.

By taking numerical values (and corresponding thresholds) into account, the results can be filtered for pathologies which are usually graded. While some values related to a code may indicate the presence of a certain finding, and, therefore, relevance for the query, other values indicate that the finding sought for is absent in the entry.

According to an aspect, the medical ontology is stored in a graph database and encodes references of codes of the medical ontology to data identifiers of the electronic medical record database, the graph database being different than the electronic medical record database. Further, parsing the electronic medical record database comprises determining pertinent data identifiers based on the encoded references and the identified at least one code and the determined related codes, and retrieving entries from the electronic medical record database based on the pertinent data identifiers.

In other words, the graph database not only comprises a data representation of the ontology but also links the ontology elements (or the codes of the ontology) to the data structure of the medical record database. This renders not only the look-up easier but also facilitates the maintenance and update of the solution. If, for instance, new medical ontology elements are included, the new elements only need to be provided with encodings pointing to the data identifiers of the medical record database; no changes to the medical record database are required.

According to some examples, the data identifiers are based on a predetermined data standard such as, for instance, the aforementioned FHIR- and/or DICOM- standards. In other words, the data identifiers are standardized, which further improves the interoperability of the method.

According to an aspect, the method further comprises determining a clinical context of the query based on the query and/or the at least one identified code, selecting a presentation state from a plurality of different predetermined presentations states based on the determined clinical context, each of the plurality of different predetermined presentation states defining a visual representation for the information, wherein the step of providing the information comprises applying the selected presentation state to the identified entries so as to generate a visual representation and displaying the visual representation.

By providing and selecting from a plurality of different presentation states, the provision of information can be adapted to the query and, therewith, to the information sought for. This improves the efficiency and useability of the method.

According to an aspect, each presentation state is associated with at least one post-processing function configured to process an entry so as to generate one or more human-interpretable observables from the entry. Further, applying the selected presentation state comprises applying the associated at least one post-processing function to the identified entries so as to generate the human-interpretable observables. Further, the visual representation is based on the generated human-interpretable observables.

According to an aspect, the method further comprises determining a clinical context of the query based on the query and/or the at least one identified code, selecting a presentation state from a plurality of different predetermined presentations states based on the determined clinical context, each presentation state defining one or more data processing steps to be executed on the identified entries for generating a distinct visual representation based on the identified entries, wherein the step of providing comprises executing the data processing steps of the selected presentation state on the identified entries so as to generate the distinct visual representation and displaying the visual representation.

By providing dedicated post processing functions, the available data can be automatically brought into a shape which increases the specificity and content of the information finally provided to the user.

Self speaking, the above aspects and examples are not only applicable to medical use cases but to the problem of searching databases, in general.

Accordingly, according to another aspect, a computer-implemented method for providing information from an electronic database is provided. The method comprises a plurality of steps. One step is directed to receiving a query for the information. Another step is directed to identifying at least one search term in the query. Another step is directed to determining one or more related search terms based on a predetermined documentation, the related search terms having a relation to the identified search term in the predetermined documentation. Another step is directed to parsing the electronic database based on the at least one identified search term and the one or more related search terms so as to retrieve the information. Another step is directed to providing the information.

The predetermined documentation may be related to the information/query. In particular, the predetermined documentation may comprise any one of a (technical) ontology, a user manual, a thesaurus database, and the like.

The further features of the other aspects and examples as discussed before may be applied to this more general case of handling data queries outside of medical applications. This applies, in particular, for the natural language processing algorithm used, the search algorithm configured to determine related search terms (codes), the ranking, the post-processing, the presentation states, the provision of a graph database and so forth.

According to an aspect, a system for providing information from an electronic medical record database comprising an interface unit and a computing unit is provided. Thereby, the computing unit is configured to receive a query for the information via the interface unit, to identify at least one code of a medical ontology on the basis of the query, to determine one or more related codes in the medical ontology based on the medical ontology and the identified at least one code, the related codes having a relation to the identified code in the medical ontology, to parse the electronic medical record database via the interface unit so as to identify entries in the electronic medical record database comprising one or more of the identified at least one code and the one or more related codes, and to providing the information on the basis of the identified entries via the interface.

The computing unit may be realized as a data processing system or as a part of a data processing system. Such a data processing system can, for example, comprise a cloudcomputing system, a computer network, a computer, a tablet computer, a smartphone and/or the like. The computing unit can comprise hardware and/or software. The hardware can comprise, for example, one or more processors, one or more memories, and combinations thereof. The one or more memories may store instructions for carrying out the method steps according to the invention. The hardware can be configurable by the software and/or be operable by the software. Generally, all units, sub-units or modules may at least temporarily be in data exchange with each other, e.g., via a network connection or respective interfaces. Consequently, individual units may be located apart from each other. Further, the computing unit may be configured as an edge device.

The interface unit may comprise an interface for data exchange with the medical records database for parsing the medical records database and receiving the entries. The interface unit may be further adapted to interface with one or more users of the system, e.g., by receiving the query and/or displaying the result of the processing, i.e., the information, to the user (e.g., in a graphical user interface).

According to an aspect, a data handling system is provided comprising the system for providing information from an electronic medical record database and the electronic medical record database.

The systems may be adapted to implement the inventive method in their various aspects for modifying medical image data. The advantages described in connection with the method aspects and examples may also be realized by the correspondingly configured systems' components. Accordingly, the advantages described in connection with the method-aspects and examples are also attributable to the corresponding systems.

According to another aspect, the present invention is directed to a computer program product comprising program elements which induce a computing unit of a system configured to provide information from an electronic medical record database to perform the steps according to one or more of the above method aspects and examples, when the program elements are loaded into a memory of the computing unit.

According to another aspect, the present invention is directed to a computer-readable medium on which program elements are stored that are readable and executable by a computing unit of a system configured to provide information from an electronic medical record database according to one or more method aspects and examples, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:

FIG. 1 schematically depicts an embodiment of a system for providing information from an electronic medical record database;
FIG. 2 schematically depicts a method for providing information from an electronic medical record database according to an embodiment;
FIG. 3 schematically depicts an exemplary data flow diagram in connection with a method for providing information (I) from an electronic medical record database according to an embodiment;
FIG. 4 schematically depicts an exemplary medical ontology according to an embodiment;
FIG. 5 schematically depicts a graphical user interface presenting provided information according to an embodiment;
FIG. 6 schematically depicts a natural language processing algorithm according to an embodiment; and
FIG. 7 schematically depicts a graph search algorithm according to an embodiment.

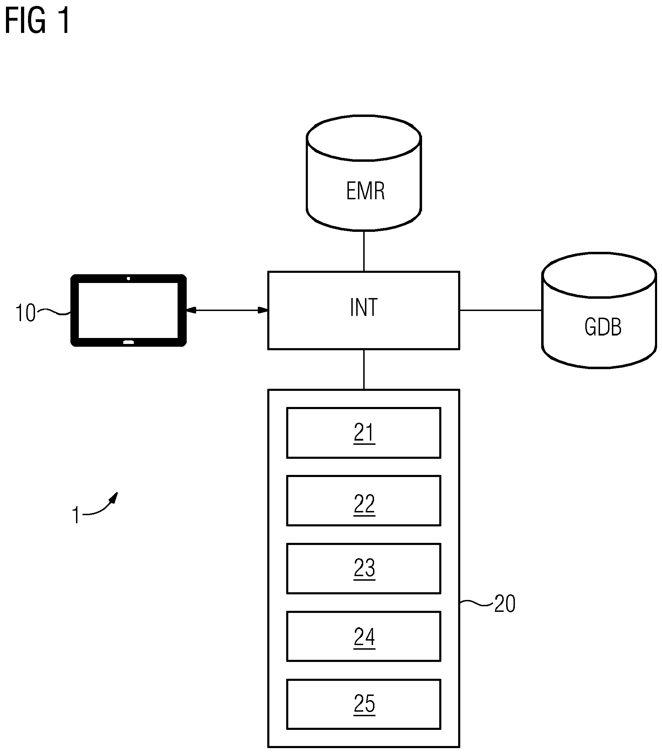

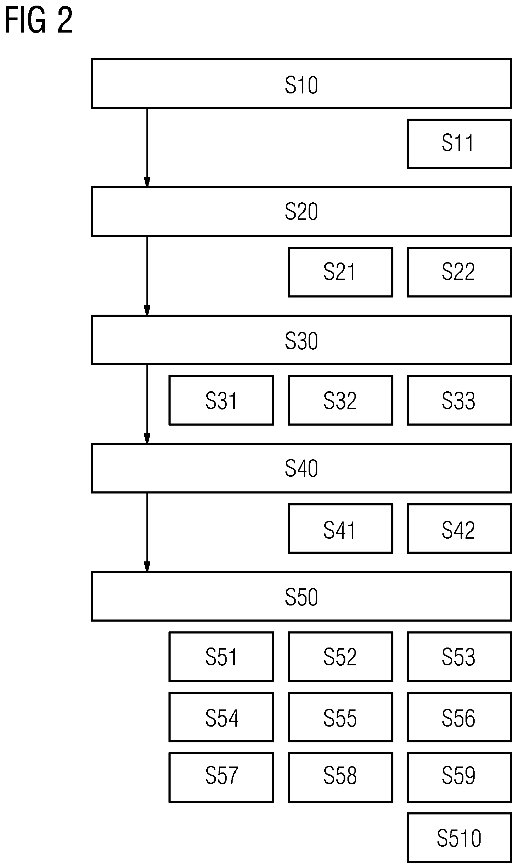

Figure 1 depicts a system 1 for providing information I from a medical record database EMR. System 1 is adapted to perform the methods according to one or more embodiments, e.g., as further described with reference to Figures 2 to 7. A user of system 1, according to some examples, may generally relate to a healthcare professional such as a physician, clinician, technician, radiologist, pathologist and so forth.

System 1 comprises a user interface 10 (as part of the interface unit) and a computing unit 20. Further, system 1 may comprise or be connected to the electronic medical record database EMR. Further, system 1 may comprise or may be connected to a medical ontology database GDB, in particular, in the form of a graph database GDB.

The electronic medical record database EMR may generally be configured for acquiring and/or storing and/or forwarding medical records of one or more patients. According to some embodiments, these records are also denoted as entries EN or studies of the respective patient. The electronic medical record database EMR may be embodied by one or more storages. In particular, the electronic medical record database EMR may be realized in the form of one or more cloud storage modules. Alternatively, the electronic medical record database EMR may be realized as a local or spread storage. According to some examples, the electronic medical record database EMR may be formatted according to a medical informatics standard such as the FHIR standard. This may mean that the electronic medical record database EMR and the entries EN therein comprised encode standardized data identifiers according to the respective standard. According to some examples, the electronic medical record database EMR is the only location where the actual patient data values are stored.

The medical entries EN may comprise medical data of a patient. For each patient registered in the electronic medical record database EMR, there may be one or more entries EN in the electronic medical record database EMR. The medical data stored in the entries EN may comprise image and non-image data.

The image data may be three-dimensional image data sets acquired, for instance, using an X-ray system, a computed tomography system or a magnetic resonance imaging system or other systems. The image information may be encoded in a three-dimensional array of m times n times p voxels. Further, image data may comprise two-dimensional medical image data with the image information being encoded in an array of m times n pixels. According to some examples, these two-dimensional image data may have been extracted from three-dimensional medical image data sets. According to other examples, two-dimensional medical image data may have been generated by dedicated imaging modalities such as slide scanners used in digital pathology.

The non-image data may be supplementary information providing additional information relating to the patient. The non-image data may relate to non-image examination results such as lab data, vital signs records (comprising, e.g., ECG data, blood pressure values, ventilation parameters, oxygen saturation levels) and so forth. Moreover, the non-image data may comprise structured and unstructured medical reports relating to prior examinations of the patient. Further, non-image data may comprise personal information of the patient such as gender, age, weight, insurance details, and so forth.

The medical ontology database GDB may generally be configured to store one or more medical ontologies MO. For instance, the medical ontology database GDB may be configured to store the medical ontologies MO in the form of a graph data structure. Thus, the medical ontology database GDB may be embodied as a graph database GDB.

The medical ontology database GDB may be embodied by one or more storages. In particular, the medical ontology database GDB may be realized in the form of one or more cloud storage modules. Alternatively, the medical ontology database GDB may be realized as a local or spread storage. Further, according to some examples, the medical ontology database GDB may be in a public domain from which the medical ontology MO can be downloaded. According to other examples, publicly available medical ontologies MO are transformed into a graph data structure which is then stored in the graph database GDB. According to other examples, natural language processing may be used to create customized ontologies MO or concept-embeddings by parsing medical corpuses. Graph vertices may be identified using a combination of ontology type, version number and ontology code.

The million+ concepts and relations may make traditional relational databases not performant to store medical ontologies MO and, hence, graph databases are used according to some examples.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen, a microphone and any combination thereof. The input unit may be configured for receiving a query Q from the user. Thereby, the query Q may be directed to retrieve a certain information I from the medical record database EMR. The query Q may contain natural language. The display unit may be configured for displaying a representation of the information I.

User interface 10 may further comprise an interface computing unit configured to execute at least one software component for serving the display unit and the input unit in order to provide a graphical user interface GUI for allowing the user to input the query Q and/or make selections affecting its further processing. In addition, the interface computing unit may be configured to communicate with the computing unit 20 for forwarding the query Q and receiving the information I. The user may activate the software component via user interface 10 and may acquire the software component, e.g., by downloading it from an internet application store. According to an example, the software component may also be a clientserver computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

Computing unit 20 may comprise sub-units 21-25 configured to process the query Q in order to adequately search or parse the electronic medical record database EMR to provide the information I sought for in the query Q.

Computing unit 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. The computing unit 20 may be comprised in the user interface 10. Alternatively, computing unit 20 may be separate from user interface 10. Computing unit 20 comprise a real or virtual group of computers like a so called 'cluster' or 'cloud'. Such server system may be a central server, e.g., a cloud server, or a local server, e.g., located on a hospital or radiology site. Further, processing system 20 may comprise a memory such as a RAM for temporally loading the medical ontology MO, relevant codes IC, RC from the medical ontology MO and/or entries EN from the medical record database EMR. According to some examples, such memory may as well be comprised in user interface 10.

Sub-unit 21 may be conceived as a natural language processing module or unit. Sub-unit 21 may be configured to convert a query Q comprising natural language (e.g., in the form of free text) to the set of ontology codes IC mentioned in that query Q. In other word, sub-unit 21 may be configured to provide at least one identified code IC of the medical ontology MO in the query Q. To identify the codes IC, sub-unit 21 may be configured to run an accordingly configured natural language processing algorithm NLPA according to some examples. The same module can also be used to create synopses of the results for more natural reading as human-interpretable object UIE.

Sub-unit 22 may be conceived as a code search module or unit configured to search the medical ontology MO for related codes RC. Sub-unit 22 may be configured to take a set of identified ontology codes IC as input and, optionally depending on a linguistic or clinical context of the query Q, select a set of search algorithms configured to determine the related codes RC. Specifically, sub-unit 22 may be configured to select and run accordingly configured graph search algorithms GSA according to some examples. To this end, sub-unit 22 may access or serve a storage or library of search methods. Such library may be a collection of plug-and-play graph search methods (hierarchical traversal, lateral traversal, definition-based traversal, etc.) which are provided as standard by graph database vendors like CosmosDB, Tigergraph, or Neo4j.

Sub-module 23 may be conceived as a data parsing or retrieval module or unit. Sub-unit 23 may be configured to search or parse the electronic medical record database EMR for entries being linked to one or more of the pertinent codes (identified codes IC as well as related codes RC). To this end, sub-unit 23 may be configured to match the codes IC, RC with data identifiers of the electronic medical record database EMR and query the EMR on that basis.

According to some examples, sub-unit 23 may comprise a data pusher module configured to take (raw) data from data sources (e.g., computer aided detection algorithms, diagnostic cockpits, image headers), map it to the ontology codes, and push it to the electronic medical record database EMR and medical ontology database GDB in a manner that the electronic medical record database EMR stores all the data values while the medical ontology database GDB only has references to (e.g., FHIR) data identifiers for each encoded data value. For instance, if the data comprises the modality code of CT (SCT_20220731_77477000) and a patient ID, e.g., P1235, the medical ontology database GDB vertex with that ID will have the following property: P1235:[Study1, Study2..] wherein Study1 is the FHIR identifier for the study that has the attribute value of CT.

Sub-unit 24 may be conceived as a post-processing module or unit. Sub-unit 24 may be configured to select and apply one or more predetermined post-pressing functions PPF to the entries EN retrieved by sub-unit 23, for instance, so as to generate one or more human-interpretable objects UIE. To this end, sub-unit 24 may access or serve a storage or library of post-processing functions PPF. This library may be seen as a collection of calculation components for a specific medical context, e.g., medical classification, in order to answer a specific query Q. This can include general post-processing functions PPF, like "sort entries EN by number of codes IC, RC comprised", "filter entries EN by value(s) included" etc. Further, this library may also include post-processing functions PPF for sorting the entries EN by appropriate relevance-scores (weights).

Sub-unit 25 may be configured as a user interaction module or unit. Sub-unit 25 may be configured to provide a graphical user interface GUI to a user for displaying to the user via the user interface 10. The graphical user interface GUI can be configured to support the presentation of the provided information I. Further, graphical user interface GUI may be configured to receive input from the user such as the query Q or selections regarding the graph search algorithms GSA or individual entries EN for further examination.

The designation of the distinct sub-units 21-25 is to be construed by way of example and not as a limitation. Accordingly, sub-units 21-25 may be integrated to form one single unit (e.g., in the form of "the computing unit") or can be embodied by computer code segments configured to execute the corresponding method steps running on a processor or the like of Computing unit 20. The same holds true with respect to the interface computing unit. Each sub-unit 21-25 and the interface computing unit may be individually connected to other sub-units and/or other components of the system 1 where data exchange is needed to perform the method steps.

Computing unit 20 and the interface computing unit together may constitute the computing unit of the system 1. Of note, the layout of this computing unit, i.e., the physical distribution of the interface computing unit and sub-units 21-25 is, in principle, arbitrary. For instance, sub-unit 21 (or individual elements of it or specific algorithm sequences) may likewise be localized in user interface 10. The same holds true for the other sub-units 21-25. Specifically, computing unit 20 may also be integrated in user interface 10. As already mentioned, computing unit 20 may alternatively be embodied as a server system, e.g., a cloud server, or a local server, e.g., located on a hospital or radiology site. According to some implementations, user interface 10 could be designated as a "frontend" or "client" facing the user, while computing unit 20 could then be conceived as a "backend" or server. Communication between user interface 10 and computing unit 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via (data) interface 26 to exchange, e.g., the query Q, the information I or any user input made. Further, computing unit 20 may communicate with electronic medical record database EMR in order to retrieve entries EN via the data interface 26. Further, computing unit 20 may communicate with medical ontology database GDB in order to retrieve the medical ontology MO via the data interface 26. Data interface 26 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 26 for data exchange together with the components for interfacing with the user be regarded as constituting an interface unit of system 1.

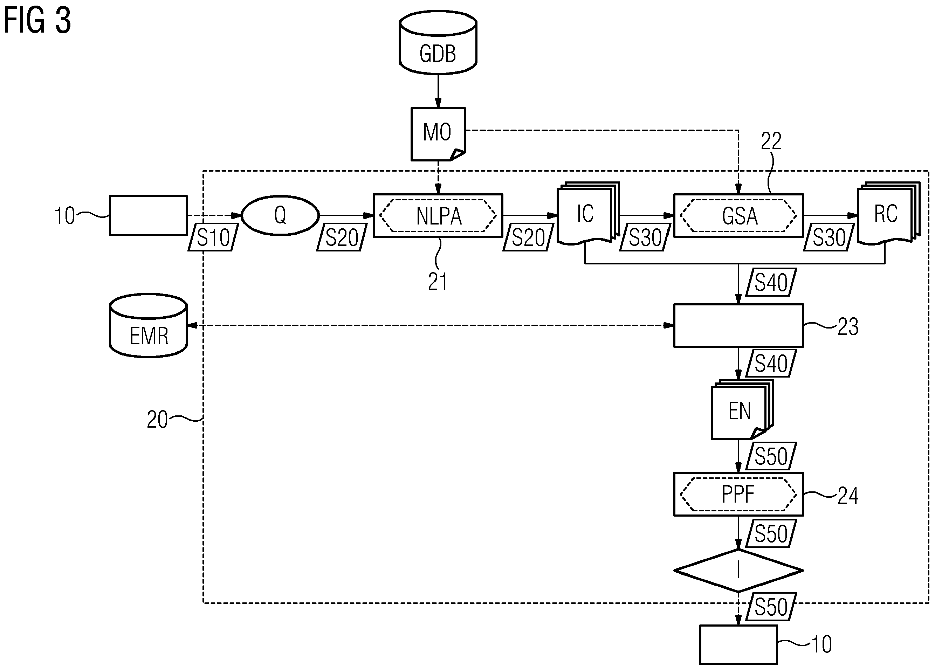

Figure 2 depicts a method for providing information I from an electronic medical record database EMR according to an embodiment. Corresponding data streams are illustrated in Figure 3. The method comprises several steps. The order of the steps does not necessarily correspond to the numbering of the steps but may also vary between different embodiments of the present invention. Further, individual steps or a sequence of steps may be repeated.

At step S10, the query Q is received. Specifically, the query Q may be received from a user of the system 1. The user may input the query Q via the user interface 10 as speech input or as text input. Accordingly, the query Q may comprise natural langue or natural language text. Any speech input may be transformed into text by a natural language recognition module (not shown) comprised in the user interface 10.

In optional step S11, a (clinical) context may be determined. The context may provide additional information as to the circumstances of the query Q and, thus, offer additional cues how to respond to the query Q correctly. For instance, the context may be determined based on the supplementary information. According to some examples, such supplementary information may be the data loaded via the user interface, the patient considered, the diagnostic task to be performed by the user, one or more suspected diseases and so forth. The supplementary information may be obtained based on the inputs made by the user in the user interface 10 or the data of the patient under consideration.

At step S20, at least on code IC of the medical ontology MO is identified in the query Q. Optionally, step S20 may comprise obtaining the medical ontology MO. This may comprise fetching the medical ontology MO from a corresponding database which may be embodied by the graph database GDB. Further, this may comprise selecting the medical ontology MO from a plurality of candidate medical ontologies. According to some examples, the selection may be based on the clinical context determined in step S11. As an alternative, a predetermined ontology may be used, such as the SNOMED ontology.

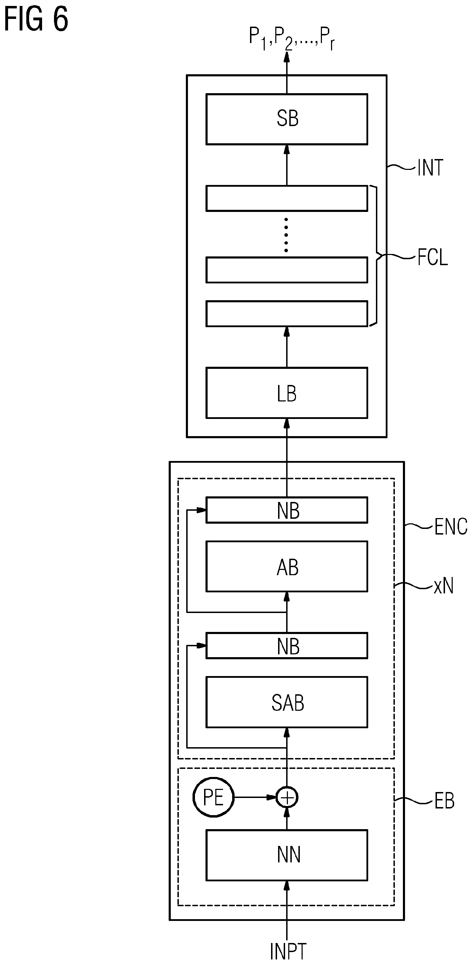

The identification of the at least one code IC may be done by a natural language processing algorithm NLPA which is configured to identify words in natural language and match these with codes of the respective codes of the medical ontology MO. The natural language processing algorithm NLPA may be rule-based. Alternatively, the natural language processing algorithm NLPA may comprise a machine learning algorithm as exemplified in Figure 6.

Step S20 may comprise the optional sub-step S21 of obtaining the natural language processing algorithm NLPA. This may involve downloading the same from a corresponding storage and/or holding it available in the computing unit 20. At step S22, the query Q is input in the natural language processing algorithm NLPA so as to identify at least one code IC indicated by the query Q. Further, the natural language processing algorithm NLPA may be configured to determine a linguistic context for each code in the query Q. The linguistic context may be used to determine the related codes RC in step S30 or to filter the information I to be provided in step S50.

At step S30 one or more related codes RC of the medical ontology MO are determined for each of the codes IC identified in step S20. The related codes RC have a predetermined relation to the respective identified code. The relation is determined by the medical ontology MO. For instance, the relation may be a positional relation, a conditional relation, or relation due to the meaning of the respective codes.

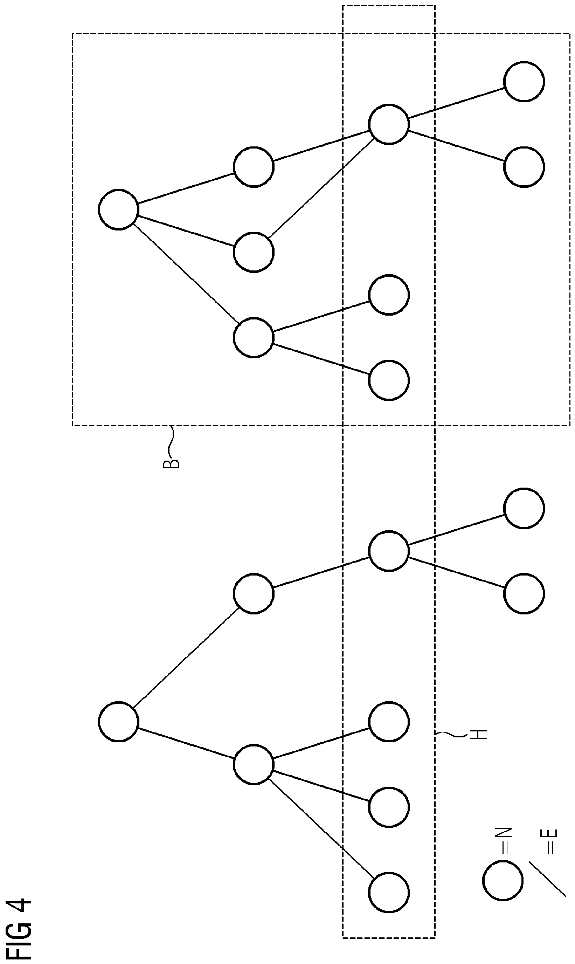

As also shown in Figure 4, a medical ontology MO may be seen as a formally ordered representation and, in particular, can be represented in the form of a mathematical graph comprising nodes N and edges E. Thereby, the codes may relate, in particular, to the nodes N and the relations based on which the related codes RC are identified may be represented by the edges E.

Accordingly, the medical ontology MO may be provided in the form of a graph data structure (optional sub-step S31). Using a graph data structure for processing the medical ontology MO has the advantage that graph search algorithms GSA may be used for exploring the medical ontology MO for determining the related codes RC.

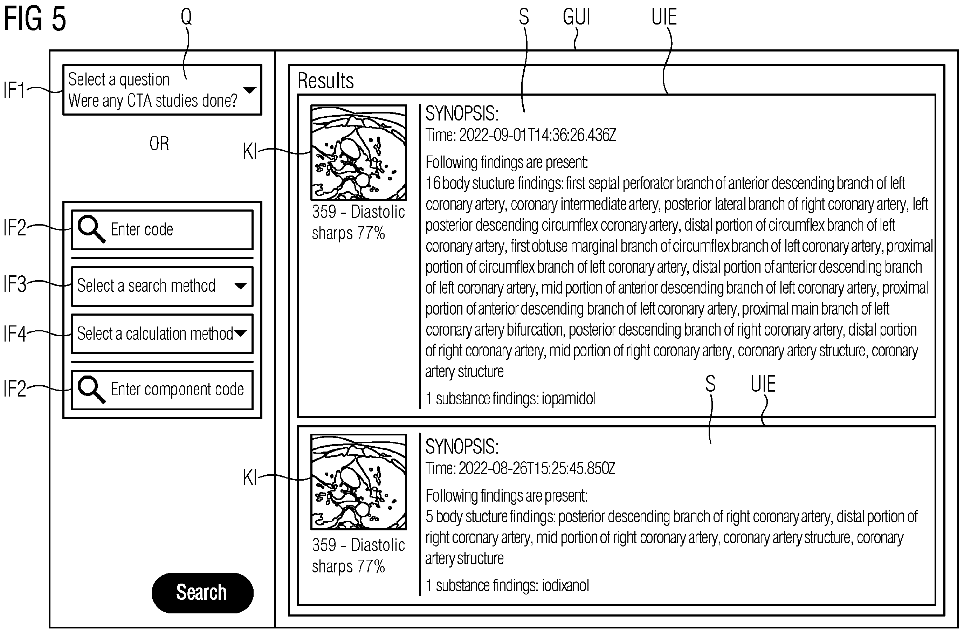

At optional sub-step S32, a graph search algorithm GSA is obtained for determining the related codes RC. This may involve selecting the graph search algorithms GSA form a plurality of candidate graph search algorithms GSA. Thereby the information available may be used for making an appropriate selection. For instance, the selection may be based on the query Q, the clinical context, the linguistic context, and/or the identified codes IC. According to other examples, the graph search algorithm GSA may be selected by the user, e.g., via a corresponding input field in the graphical user interface GUI(see Figure 5).

At optional sub-step S33, the medical ontology MO and the identified codes IC are input into the graph search algorithm GSA so as to determine the related codes RC.

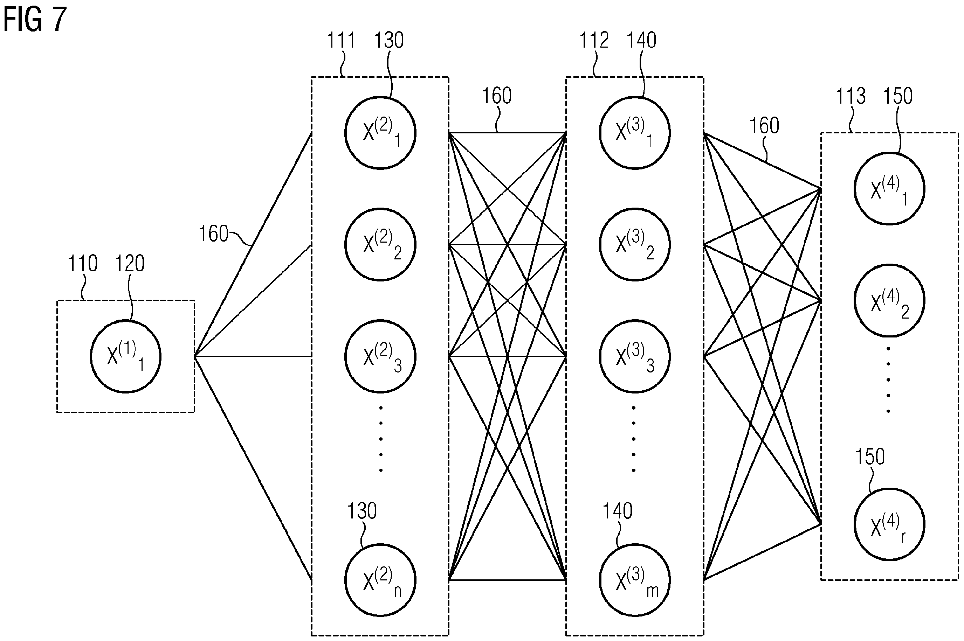

The graph search algorithm GSA is generally configured to determine related codes RC in a medical ontology MO. The graph search algorithm GSA may implement various different search strategies to parse the medical ontology MO. The graph search algorithm may be rule based in the sense that it follows a predefined search pattern for determining related codes. Further, the graph search algorithm GSA may comprise a machine learned function which has been trained to output related codes RC if provided with a medical ontology MO and input codes IC. According to an embodiment, such a machine learned function may comprise a neural network. An exemplary embodiment is shown in Figure 7.

At step S40 the electronic medical record database EMR is searched for entries EN comprising or at least indicating one or more of either the identified codes IC or the related codes RC.

According to an embodiment, the method again may make use of the data structure of the medical ontology MO and the standard format of the data entries EN in the medical record database EMR. Specifically, the medical ontology MO or rather its data structure may be provided with pointers or references from the codes to corresponding data identifiers of the standard format of the electronic medical record database EMR. Accordingly, at optional sub-step S41, the pointers of the identified codes IC and the related codes RC may be identified. On that basis, the corresponding data identifiers of the electronic medical record database EMR may be determined. That followed, these data identifiers may be used to query the electronic medical record database EMR. Specifically, the electronic medical record database EMR may be searched for entries having the determined data identifiers (at optional sub-step S42).

At step S50 the information I is compiled based on the entries EN retrieved at step S40. Further, the information I is provided at step S50. This may involve displaying the information I in the user interface 10, for instance, in the form of a graphical user interface GUI. As will be outlined in the following, this may involve several post-processing steps of the data comprised in the retrieved entries EN. The following steps may be performed individually or in arbitrary combinations.

At optional sub-step S51, a ranking of the entries EN is determined based on the identified codes IC and the related codes RC. Thereby, the ranking may be such that an entry ranks higher, the more instances of the codes IC, RC (identified or related) can be identified in the entry EN. Further, the ranking may also be based on the codes IC, RC as such, e.g., on their meaning/significance for the query Q. For instance, the codes IC, RC may be provided with a weight so that codes IC, RC with a higher weight lead to a comparable higher ranking of the entry EN than codes with a lower weight. According to some examples, the weights may be assigned based on the medical ontology MO. For instance, the weights for the related codes RC may decrease with the distance to the identified codes IC in the medical ontology MO.

At optional sub-step S52, the information I is displayed in the user interface 10 based on the ranking. For instance, entries EN ranking higher may show up on top of a list of results making up the provided information I.

At optional sub-step S53, a post-processing function PPF is provided. The post-processing function PPF is generally configured to process the data comprised in the retrieved entries EN so as to compile the information I to be provided. For instance, the post-processing function PPF may be configured to extract data items from the entries EN such as individual numbers or images. According to other examples, the post-processing function PPF comprises a filter function configured to filter those entries EN from the retrieved entries EN that match the query Q. For instance, such a filter function may be configured to search for a data pattern in the retrieved entries EN. According to some examples, the post-processing function PPF comprises a classification function configured to classify the entries EN according to one or more clinical states. Then, only entries EN classified according to particular clinical states may be considered for providing the information I.

Providing the post-processing function PPF at step S53 may comprise selecting the post-processing function PPF from a plurality of candidate post-processing functions based on the query Q, the clinical context, the linguistic context, and/or the identified code(s) IC.

At optional sub-step S54, the post-processing function PPF is applied to the retrieved entries EN so as to generate the information I. The post-processing function PPF may be configured to generate a human-interpretable object UIE from the retrieved entries EN. The human-interpretable object UIE may, for instance, comprise a number, a chart or trending graph, an image, a synopsis, and so forth.

At optional sub-step S55, the human-interpretable object UIE is provided. According to some examples, the human-interpretable object UIE may be an element in the graphical user interface GUI (c.f., Figure 5 for an example).

At optional sub-step S56, a synopsis S is generated. Specifically, a synopsis S may be generated for each retrieved entry EN. According to some examples, the synopsis S may be generated by applying an accordingly configured post-processing function PPF. Specifically, such post-processing function PPF may comprise a natural language processing algorithm configured to generate a synopsis S based on an entry EN of the electronic medical record database EMR. The natural language processing algorithm may be of the same kind as the example of Figure 6.

At optional sub-step S57, the synopsis S is provided, for instance, in the form of a human-interpretable object UIE as described above.

At optional sub-step S58, a threshold for a numerical value is determined. The numerical value is related to a one of the identified codes IC and the related codes RC. The threshold may indicate if an entry falls into a predetermined category. At step S58, this threshold may be determined based on the query Q, the clinical context, the linguistic context, and/or the identified code IC. Establishing the threshold may be performed by an accordingly configured post-processing function PPF.

At optional sub-step S59, the entries EN are selected based on the threshold. For instance, entries EN the numerical values of which are below the threshold may be disregarded. That followed only the selected entries EN may be used for generating and providing the information I. Likewise, any (additional) post-processing function PPF may only be applied to the selected entries EN.

At optional sub-step S510, a presentation state may be provided which determines what data is extracted from the entries EN, how this data is processed, and/or how the thus processed data is presented to provide the information I. For instance, a presentation state me define the graphical user interface GUI and the human-interpretable objects UIE. In turn, the presentation state may define the post-processing functions PPF to be applied to the retrieved entries EN so as to generate the human-interpretable objects UIE.

According to some examples, providing the presentation state may comprise selecting the presentation state from a plurality of candidate presentation states based on the query Q, the clinical context, the linguistic context, and/or the identified code(s) IC. The candidate presentation states may be pre-configured for different kinds of queries Q, clinical contexts, linguistic contexts, and/or identified codes IC.

That followed, the presentation state may be applied to the retrieved entries EN so as to generate the information I to be provided.

Figure 4 schematically shows an exemplary medical ontology MO according to an embodiment.

As shown in Figure 4, a medical ontology MO may be seen as a formally ordered representation of a set of elements and the relationships between them. In particular, the elements of a medical ontology MO may relate to medical concepts, medical data and/or medical information. The medical ontology MO can be represented, in particular, in the form of a mathematical graph comprising nodes N and edges E, in particular in the form of a directed graph. According to some examples, the nodes N and/or the edges E can, in particular, have further data such as numerical values.

According to some examples, the nodes N of the medical ontology MO may be associated with the codes as herein described, or, in other words, each code may refer to a node N of the medical ontology MO. The edges E of the medical ontology MO may define the relations of the nodes N and, therewith the relations of the codes of the medical ontology MO. Thus, for an arbitrary code IC, related codes RC may be determined based on the edges E of the node N corresponding to the arbitrary code IC.

As can be seen in Figure 4, the nodes N may be ordered in branches B with individual nodes N branching off an hierarchically higher node N. Thus, the medical ontology MO may define a system of different hierarchy levels H of nodes N within the branches B of the medical ontology MO and neighboring branches B. Switching between branches B at substantially the same hierarchy level H may be equated with moving laterally in the medical ontology MO. Moving within a branch B over the different hierarchy levels H may be equated with hierarchal traversing the medical ontology MO.

Figure 5 shows a graphical user interface GUI according to an embodiment. The graphical user interface GUI is an example how the information I can presented in the user interface 10. Further, the graphical user interface GUI exemplarily shows how the retrieved entries EN or, rather, the data therein comprised, are processed to provide the information I.

As explained, the graphical user interface GUI may be based on a distinct presentation state which, in turn, defines one or more post-processing functions PPF configured to process data comprised in the entries EN. Specifically, the post-processing functions PPF may lead to human-interpretable objects UIE that are integrated in the graphical user interface GUI. As shown in Figure 5, the human-interpretable objects UIE may comprise a synopsis S and/or a key image KI or the like.

According to some examples, the human-interpretable objects UIE may be ranked. The ranking may be based on the underlying entries EN. In the example of Figure 5, each human-interpretable object UIE stands for an entry EN.

Further, as shown in Figure 5, the graphical user interface GUI may comprise an input field IF1 for inputting the query Q. Further, according to some examples, the graphical user interface GUI may comprise an input field IF2 for specifying or modifying an identified code IC of the medical ontology MO directly by the user. Further, according to some examples, the graphical user interface GUI may comprise an input field IF3 for specifying the search method, e.g., by selecting the graph search algorithm GSA to be applied. Further, according to some examples, the graphical user interface GUI may comprise an input field IF4 for selecting one of the post-processing functions PPF.

In Figure 6, a schematic representation of the natural language processing algorithm NLPA is shown. The natural processing algorithm NLPA of this embodiment is implemented as a transformer network or model TM. The transformer network TM is configured to process the corpus of text in the query Q as input INPT and return indications $P_1, P_2,..., P_r$ that the query Q comprises (or refers to) one or more of a set of r predetermined codes IC of the medical ontology MO.

On a high level, the transformer network TM is configured to parse the input text and detect indications for the presence or absence of one of the predetermined codes of the medical ontology MO. To fulfill this task, the transformer network has, in particular, learned the ability to deal with synonyms and semantic contexts such as negations or affirmations.

The transformer network TM according to this embodiment has an encoder ENC and an interpreter INT. In brief, the task of the encoder ENC is to map the input INPT to a sequence of continuous representations R, which is then fed into the interpreter INT. The interpreter INT then maps the representations R to the desired output - in the present case the indications $P_1$, $P_2$,..., $P_r$.

As shown in Figure 6, the encoder ENC comprises a plurality of blocks, which may respectively be formed by one or more layers of a neural network.

A first block is configured as embedding block EB. The embedding block EB is configured to transform every word comprised in the input text into a machine-readable format, the so-called input embeddings. Input embeddings, in particular, are real-valued vectors representing the underlying word in the input INPT. In this example, the vectors may have a dimension of 768, that is, each vector of the input embeddings may have 768 entries. Accordingly, the embedding block may have 768 output nodes. The input embeddings encode the meaning of the word such that words that are closer in the vector space are expected to be similar in meaning. As mentioned, a neural network NN may be used to generate the input embeddings, according to some examples. Specifically, known word-to-vector models may be used as, for instance, described in Mikolov et al., "Efficient Estimation of Word Representations in Vector Space" in arXiv:1301.3781v3, September 7, 2013, the contents of are herein included by reference in their entirety.

Further, in order to capture information about the relative positions of the words in the input INPT, the embedding block EB may be configured to generate positional encodings PE for the input embeddings. The positional encodings PE are of the same dimension as the input embeddings and may be generated using sine and cosine functions of different frequencies. Then, the positional encodings PE may be simply summed to the input embeddings in order to inject the positional information PE in the input embeddings.

The thus generated and modified input-embeddings are input in encoder ENC. The encoder ENC of this embodiment may comprise a stack of N=6 identical blocks. For the sake of easy reference, only one block xN is shown in the drawing. Further, N may also be set to different values and, in particular, to values greater than N=6, according to the respective task. Each block xN of the encoder ENC comprises two subblocks SAB and AB. The first subblock SAB implements a so-called self-attention mechanism. Specifically, the first subblock SAB may be configured to determine how relevant a particular word or input embedding is with regard to other words or input embeddings in the input INPT. This may be represented as an attention vector. In some examples, the first subblock SAB may be configured to compare 64 words yielding a correspondingly dimensioned attention vector per word. To avoid any bias, multiple attention vectors per word may be generated and fed into a weighted average to compute the final attention vector of every word. There are various ways how the attention mechanism can be implemented in a neural network as, for instance, described in Vaswani et al., "Attention Is All You Need", in arXiv: 1706.03762, June 12, 2017, the contents of which are herein included by reference in their entirety.

The second subblock AB is an adaptation block configured to bring the output of the self-attention subblock SAB into a suitable format for the subsequent processing. The adaptation block AB may comprise a fully connected feed-forward network which may, for example, comprise two linear transformations with Rectified Linear Unit (ReLU) activation in between.

The N=6 layers of the encoder ENC apply the same linear transformations to all the words in the input INPT, but each layer employs different weight and bias parameters to do so. Each subblock SAB, AB is succeeded by a normalization block NB, which normalizes the sum computed between the input fed into the respective subblock SAB, AB and the output generated by the respective subblock SAB, AB itself.

After all layers xN of the encoder ENC have been processed, the results are fed into the interpreter INT. The interpreter INT may comprise a linear block LB which may be another feed-forward layer. It is used to expand the dimensions into a format expected for computing the output vector OUT. That followed, the result is parsed through a plurality of fully connected layers FCL which successively map the processing result of the encoder ENC to the space of output values. Specifically, the last fully connected layer may comprise as many nodes as there are codes to be considered, in order to provide the indications $P_1$, $P_2$, ..., $P_r$. Accordingly, the last fully connected layer generally has r output nodes. Finally, the result of the last fully connected layer may be passed through a softmax block SB for obtaining normalized indications of either 1 or 0.

For training the transformer model TM, a pre-trained encoder ENC is provided. Pre-trained encoders ENC are available for various languages. One advantage of using pre-trained encoders is that they already know the basic semantics of the language the transformer model TM will have to deal with. For an ensuing end-to-end training of the transformer model TM according to the present task of identifying the comorbidity indications $P_1$, $P_2$, ..., $P_r$, a database of 1000 entries of a medical record database EMR annotated by expert physicians was used. In particular, the experts determined for each of the 1000 datasets whether the respective entry refers to a certain code of the medical ontology MO, indicated by 0 or 1 (ground truth). The database was split into training data (640 datasets), validation data (160 datasets) and test data (200 datasets). For training the transformer model, the backpropagation algorithm was used based on a loss function $L(INPT, y_1, y_2, ..., y_r) = |M(INPT)_1 - y_1|^2 + |M(INPT)_2 - y_2|^2 + ... + |M(INPT)_r - y_r|^2$, wherein x denotes an input medical report, $y_1$, $y_2$, ..., $y_r$ denote whether the respective query Q has one of the pre-determined codes (as evaluated by the expert). Furthermore, M(INPT) denotes the result of applying the transformer model TM to the input medical report INPT, and $M(INPT)_1$, $M(INPT)_2$, ... , $M(INPT)_r$ correspond to the value of the respective output node of the interpreter INT if applying the machine learning model to the input medical report INPT.

Based on the validation set of 160 datasets and the corresponding annotations, the best performing transformer model TM out of several transformer models (with different hyperparameters, e.g., number of fully connected layers, stack size N, embedding block EB etc.) was selected. The specificity and the sensitivity were determined based on the test set comprising 200 datasets and the corresponding annotations.

In Figure 7 an exemplary realization of a graph-search algorithm GSA according to an embodiment is shown. In this embodiment, the graph-search algorithm GSA comprises a neural network 100 as a trained function.

The neural network 100 comprises network nodes 120, ..., 150 and network edges 160 wherein each network edge 160 is a directed connection from a first network node 120, ..., 150 to a second network node 120, ..., 150. In general, the first network node 120, ..., 150 and the second network node 120, ..., 150 are different network nodes 120, ..., 150, it is also possible that the first network node 120, ..., 150 and the second network node 120, ..., 150 are identical.

In this embodiment, the network nodes 120, ..., 150 of the neural network 100 can be arranged in layers 110, ..., 113, wherein the layers can comprise an intrinsic order introduced by the network edges 160 between the network nodes 120, ..., 150. In particular, network edges 160 can exist only between neighboring layers of nodes. In the displayed embodiment, there is an input layer 110 comprising only network nodes 120 without an incoming edge, an output layer 113 comprising only network nodes 150 without outgoing edges, and hidden layers 111, 112 in-between the input layer 110 and the output layer 113. In general, the number of hidden layers 111, 112 can be chosen arbitrarily. The number of network nodes 120, ..., 150 within the input layer 110 usually relates to the number of input values of the neural network, and the number of network nodes 150 within the output layer 113 usually relates to the number of output values of the neural network.

In particular, a (real) number can be assigned as a value to every network node 120, ..., 150 of the neural network 100. Here, $x^{(n)}_i$ denotes the value of the i-th network node 120, ..., 150 of the n-th layer 110, ..., 113. The values of the network nodes 120 of the input layer 110 are equivalent to the input values of the neural network 100, the values of the network nodes 150 of the output layer 113 are equivalent to the output value of the neural network 100. Furthermore, each network edge 160 can comprise a weight being a real number, in particular, the weight is a real number within the interval [-1, 1] or within the interval [0, 1] . Here, $w^{(m,n)}_{i,j}$ denotes the weight of the network edge between the i-th node 120, ..., 150 of the m-th layer 110, ..., 113 and the j-th node 120, ..., 150 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 150 of the (n+1)-th layer 110, ..., 113 can be calculated based on the values of the nodes 120, ..., 150 of the n-th layer 110, ..., 113 by

$$x^{(n+1)}_j = f\left(\sum_i x^{(n)}_i \cdot w^{(n)}_{i,j}\right)$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network 100, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the network edges, the neural network 100 has to be trained using training data. In particular, training data comprises training input data and training output data. For a training step, the neural network 100 is applied to the training input data to generate calculated output data. In particular, the training data and the calculated output data comprise a number of values, said number being equal with the number of network nodes of the output layer.

In particular, a comparison between the calculated output data and the training data is used to re-cursively adapt the weights within the neural network 100 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be re-cursively calculated as

$$\delta^{(n)}_{j} = (\Sigma_k \; \delta^{(n+1)}_{k} \cdot w^{(n+1)}_{j,k}) \cdot f' \; (x^{(n)}_{i} \cdot w^{(n)}_{i,j})$$

based on $\delta^{(n+1)}_{j}$, if the (n+1)-th layer is not the output layer, and

$$\delta^{(n)}_{j} = (x^{(n+1)}_{j} - t^{(n+1)}_{j}) \cdot f' \; (x^{(n)}_{i} \cdot w^{(n)}_{i,j})$$

if the (n+1)-th layer is the output layer 113, wherein f' is the first derivative of the activation function, and $t^{(n+1)}_{j}$ is the comparison training value for the j-th node of the output layer 113.

The neural network 100 displayed in Figure 7 can be used for predicting related codes RC in a medical ontology MO with respect to an input code IC. The neural network 100 takes as input the identified code(s) IC. By training, the neural network 100 is configured to determine related nodes RC in the medical ontology MO for broadening the search space for the ensuing parsing of the electronic medical record database EMR.

For this embodiment, a sigmoid transfer function is used for transferring the input code IC to a real number in the range between 0 and 1 for the input node 120. The network may have as many output nodes 150 as there are codes in the medical ontology MO. The values of the output nodes 150 are interpreted as the probability for a certain code to be related to the input code IC

For the purpose of training the neural network 100, a retrospective assignment of related codes RC to various input codes IC was performed. The assigned related codes were compared to the predicted related codes RC as output by the network. On the basis of the comparison, the weights of the neural network 100 were adjusted.

In the following, two working examples for the workflow are given. The technology stack used in the examples included an Azure App for front-end (as the user interface 10) and backend (as the computing unit 20) deployments, a Cosmos SQL database for FHIR (as the electronic medical record database EMR), a Cosmos Gremlin database for graphs (as the medical ontology database or graph database GDB), and python+JS-based scripts for programming the workflow (such as search and data retrieval steps). Further, rule-based search schemes were used as graph search algorithms GSA. For the natural language processing algorithm NLPA, a custom developed algorithm based on a standard transformer network (see Figure 6) was used. The main ontology MO used was SNOMED v20220731 as it has inherent relations between the concepts which can be leveraged for determining related codes RC. However, this is to be construed by way of example and not as limiting the disclosure.

The query Q according to example 1 is "Were any prior CTAs done?".

In a first step, the following code IC is identified by the natural language processing algorithm NLPA: SCT_20220731_418272005 (which is the SNOMED-code for "CTA").

In a second step, "recursive lateral" and, that followed, "hierarchical" graph search algorithms GSA were selected. In other words, a breadth-first search scheme is selected.

In a third step, the graph database GDB encoding the SNOMED medical ontology MO is searched accordingly.

With that, in a fourth step, the SNOMED codes for CT (SCT_20220731_77477000 - the parent of "CTA"), blood vessel structure (SCT_20220731_59820001 - the procedure site of "CTA") and contrast media (SCT_20220731_385420005 - as the substance attribute for "CTA") are identified as laterally related codes IC.

In a fifth step, the hierarchical recursive search on these codes is performed and finds references at 18 segment codes of the blood vessel structure (which are children of blood vessel structure code), at the iopamidol code (which is a child of the contrast media code) and, again, at the CT-code (which is a parent of CTA) .

In a sixth step, a FHIR-lookup is performed based on the codes thus retrieved (identified codes IC and related codes RC) for the respective patient identifier. With that, pertinent entries EN are retrieved.

In a seventh step, post-processing functions PPF are applied to these entries EN. According to this example, the post-processing function PPF may be configured to rank the retrieved entries. As an example, the entries EN are returned as list of entries EN in decreasing number of found codes IC, RC (or FHIR identifiers corresponding to the codes). If, for instance, two entries EN are returned, one study had findings on the 18 segment codes and the contrast media is also mentioned. The other entry EN had only segment-level findings. Hence, the more relevant entry EN is the first one as it satisfied the most criteria in the form of the derived codes IC, RC. An exemplary output in the form of a graphical user interface GUI is shown in Figure 5.

Of note, the code "CTA" is not present in the FHIR data standard, meaning that there is no corresponding identifier. Thus, directly querying the electronic medical record database EMR would not have yielded any hits. By broadening the search space using the medial ontology MO, however, appropriate search results could still be returned.

The query Q of example 2 is "Are there any true bifurcation lesions?". Also in this example, the data source EMR has no concept for bifurcation lesions. Hence a direct search will not reveal any results. Still, the method and systems as herein disclosed are able to give relevant results based on the following exemplary steps:

In a first step, the natural language processing algorithm NLPA identifies the SNOMED codes IC of seven different bifurcation lesions (e.g., LCA bifurcation SCT_20220731_1220633009) based on the query Q.

In a second, step a graph search algorithm GSA implementing a "definition lateral" search scheme was selected.

In a third step, the SNOMED medical ontology MO is searched on that basis, i.e., using the identified codes IC and the selected graph search algorithm GSA.

In a fourth step, the related SNOMED codes RC are determined. For instance, the LCA bifurcation SCT_20220731_ 1220633009-code has four children pertaining to SNOMED-codes for LM, pLAD, pCX and RI.

In a sixth step, the FHIR-identifiers for the thus retrieved codes IC, RC are determined.

In a seventh step, a FHIR-lookup is done in the electronic medical record database EMR based on these identifiers for the specific patient (i.e., with the specific patient identifier).

In an eight step, post-processing functions PPF are applied to the entries EN. According to this example, one post-processing function PPF is applied to detect if a numerical value associated with the SNOMED codes for the bifurcation are above a certain threshold. Specifically, it is checked if at least two segments of each bifurcation have a maximal stenosis severity of 20. If so, it is classified as true bifurcation lesion. This post-processing function PPF is, in other words, configured to fetch the required value from the retrieved FHIR identifiers, do the check, and return the results

At a ninth step, the different bifurcation lesions and the segments that satisfied its classification (i.e., which are above the threshold) are returned to the user, e.g., in the graphical user interface GUI.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

Independent of the grammatical term usage, individuals with male, female or other gender identities are included within the term.

**Claims**

1. Computer-implemented method for providing information (I) from an electronic medical record database (EMR), the method comprising the following steps:

   - receiving (S10) a query (Q) for the information (I),
   - identifying (S20) at least one code (IC) of a medical ontology (MO) on the basis of the query (Q),
   - determining (S30) one or more related codes (RC) in the medical ontology (MO) based on the medical ontology (MO) and the identified at least one code (IC), the one or more related codes (RC),
   - parsing (S40) the electronic medical record database (EMR) so as to identify one or more entries (EN) in the electronic medical record database (EMR) indicating one or more of the identified at least one code (IC) and the one or more related codes (RC), and
   - providing (S50) the information (I) on the basis of the identified one or more entries (EN).

2. Method according to claim 1, wherein the query (Q) comprises natural language and wherein the step of identifying (S20) at least one code (IC)comprises:

   - providing (S21) a natural language processing algorithm (NLPA) configured to identify codes (IC) of the medical ontology (MO) in natural language, and
   - inputting (S22) the query (Q) in the natural language processing algorithm (NLPA) so as to identify the at least one identified code (IC) of the medical ontology (MO) in the query (Q).

3. Method according to any one of claims 1 or 2, wherein the step of determining (S30) one or more related codes (RC) comprises:

   - obtaining (S31) the medical ontology (MO) as a graph data structure,
   - obtaining (S32) a graph search algorithm (GSA) configured to determine related codes in graph data structures,
   - inputting (S33) the medical ontology (MO) and the at least one identified code (IC) in the graph search algorithm (GSA) so as to determine the one or more related codes (RC).

4. Method according to claim 3, wherein the step of obtaining (S32) the graph search algorithm (GSA) comprises:

   - providing a plurality of different graph search algorithms, and
   - selecting the graph search algorithm (GSA) from the plurality of different graph search algorithms based on the at least one identified code (IC) and/or a type of the medical ontology (MO) and/or the query (Q).

5. Method according to claim 4, wherein the plurality of graph search algorithms comprises a first graph search algorithm implementing a depth-first search scheme or a hierarchical recursive search scheme, and a second graph search algorithm implementing a breadth-first search scheme or a lateral recursive search scheme.

6. Method according to any one of the preceding claims, further comprising

   - ranking (S51) the entries (EN) based on the number of references to the at least one identified code (IC) and to the number of references to the one or more related codes (RC) respectively comprised in each entry (EN), wherein
   - the step of providing (S50) comprises displaying (S52) the entries according to the ranking.

7. Method according to any one of the preceding claims, wherein the step of providing (S50) comprises:

   - providing (S53) a post processing function (PPF) configured to process the entries (EN), optionally based on one or more of the at least one identified code (IC) and the one or more related codes (RC) and/or the query (Q), so as to generate one or more human-interpretable observables (UIE),
   - applying (S54) the post-processing function (PPF) to the entries (EN),
   - providing (S55) the human-interpretable observables (UIE) .

8. Method according to any one of the preceding claims, further comprising,

   - generating (S56) a synopsis (SY) for each entry (EN), optionally based on one or more of the at least one identified code (IC) and the one or more related codes (RC) and/or the query (Q), wherein
   - the step of providing (S50) comprises providing (S57) the generated synopses (SY).

9. Method according to any one of the preceding claims, wherein

   - at least one of the at least one identified code (IC) and the one or more related codes (RC) is associated with a numerical value,

   further comprising:

   - determining (S58) a threshold for the numerical value based on the query (Q), wherein,
   - in the step of providing (S50), entries are selected (S59) from the identified entries (EN) based on the threshold and respective numerical values, wherein the information (I) is based on the selected entries (EN).

10. Method according to any one of the preceding claims,

    - wherein the medical ontology (MO) is stored in a graph database (GDB) and encodes references of codes of the medical ontology (MO) to data identifiers of the electronic medical record database (EMR), the graph database (GDB) being different than the electronic medical record database (EMR), and
    - wherein parsing (S40) the electronic medical record database (EMR) comprises:

      determining (S41) at least one pertinent data identifier based on the encoded references and the at least one identified code (IC) and the one or more related codes (RC), and
      retrieving (S42) entries from the electronic medical record database (EMR) based on the at least one pertinent data identifier.

11. Method according to any one of the preceding claims, further comprising:

    - determining (S11) a clinical context of the query (Q) based on the query (Q) and/or the at least one identified code (IC),
    - selecting (S510) a presentation state from a plurality of different predetermined presentations states based on the determined clinical context, each of the plurality of different predetermined presentation states defining a visual representation for the information (I),
wherein the step of providing (S50) the information (I) comprises applying the selected presentation state to the identified entries so as to generate a visual representation (GUI) and displaying the visual representation (GUI).

12. Method according to claim 11, wherein

 - each presentation state is associated with at least one post-processing function (PPF) configured to process an entry (EN) so as to generate one or more human-interpretable observables (UIE) from the entry,

     applying the selected presentation state comprises applying the associated at least one post-processing function (PPF) to the identified entries (EN) so as to generate the human-interpretable observables, and the visual representation (GUI) is based on the generated human-interpretable observables.

13. System (1) for providing information from an electronic medical record database (EMR) comprising an interface unit (10) and a computing unit (20), wherein the computing unit (20) is configured to:

     - receive (S10) a query (Q) for the information (I) via the interface unit (10),
     - identify (S20) at least one code (IC) of a medical ontology (MO) on the basis of the query (Q),
     - determine (S30) one or more related codes (RC) in the medical ontology (MO) based on the medical ontology (MO) and the at least one identified code (IC),
     - parse (S40) the electronic medical record database (EMR) via the interface unit (10) so as to identify entries (EN) in the electronic medical record database (EMR) comprising one or more of the at least one identified code (IC) and the one or more related codes (RC), and
     - provide (S50) the information (I) on the basis of the identified entries (EN) via the interface unit (10).

14. Computer program product comprising program elements which induce a computing unit (20) of a system (1) for providing information (I) from an electronic medical record database (EMR)to perform steps of the method according to any of claims 1 to 12 when the program elements are loaded into a memory of the computing unit (20).

15. Computer-readable medium on which program elements are stored that are readable and executable by a computing unit (20) of a system (1) for providing information (I) from an electronic medical record database (EMR) to perform steps of the method according to any of claims 1 to 12 when the program elements are executed by the computing unit (20).

FIG 1

FIG 2

```
┌─────────────────────────────────────────────┐
│                    S10                        │
└─────────────────────────────────────────────┘
          │                      ┌──────────┐
          │                      │   S11    │
          │                      └──────────┘
          ▼
┌─────────────────────────────────────────────┐
│                    S20                        │
└─────────────────────────────────────────────┘
          │            ┌──────────┐ ┌──────────┐
          │            │   S21    │ │   S22    │
          │            └──────────┘ └──────────┘
          ▼
┌─────────────────────────────────────────────┐
│                    S30                        │
└─────────────────────────────────────────────┘
          │   ┌──────────┐ ┌──────────┐ ┌──────────┐
          │   │   S31    │ │   S32    │ │   S33    │
          │   └──────────┘ └──────────┘ └──────────┘
          ▼
┌─────────────────────────────────────────────┐
│                    S40                        │
└─────────────────────────────────────────────┘
          │            ┌──────────┐ ┌──────────┐
          │            │   S41    │ │   S42    │
          │            └──────────┘ └──────────┘
          ▼
┌─────────────────────────────────────────────┐
│                    S50                        │
└─────────────────────────────────────────────┘
     ┌──────────┐ ┌──────────┐ ┌──────────┐
     │   S51    │ │   S52    │ │   S53    │
     └──────────┘ └──────────┘ └──────────┘
     ┌──────────┐ ┌──────────┐ ┌──────────┐
     │   S54    │ │   S55    │ │   S56    │
     └──────────┘ └──────────┘ └──────────┘
     ┌──────────┐ ┌──────────┐ ┌──────────┐
     │   S57    │ │   S58    │ │   S59    │
     └──────────┘ └──────────┘ └──────────┘
                               ┌──────────┐
                               │   S510   │
                               └──────────┘
```

FIG 3

EP 4 376 014 A1

FIG 4

# FIG 5

**Q** — Select a question: Were any CTA studies done?

**IF1** · **IF2** (Enter code) · **IF3** (Select a search method) · **IF4** (Select a calculation method) · **IF2** (Enter component code)

OR

**Search**

**S · GUI · UIE**

## Results

**KI** — 359 - Diastolic sharps 77%

SYNOPSIS:
Time: 2022-09-01T14:36:26.436Z

Following findings are present:
16 body stucture findings: first septal perforator branch of anterior descending branch of left coronary artery, coronary intermediate artery, posterior lateral branch of right coronary artery, left posterior descending circumflex coronary artery, distal portion of circumflex branch of left coronary artery, first obtuse marginal branch of circumflex branch of left coronary artery, proximal portion of circumflex branch of left coronary artery, distal portion of anterior descending branch of left coronary artery, mid portion of anterior descending branch of left coronary artery, proximal portion of anterior descending branch of left coronary artery, proximal main branch of left coronary artery bifurcation, posterior descending branch of right coronary artery, distal portion of right coronary artery, mid portion of right coronary artery, coronary artery structure, coronary artery structure

1 substance findings: iopamidol

**S · UIE**

**KI** — 359 - Diastolic sharps 77%

SYNOPSIS:
Time: 2022-08-26T15:25:45.850Z

Following findings are present:
5 body stucture findings: posterior descending branch of right coronary artery, distal portion of right coronary artery, mid portion of right coronary artery, coronary artery structure, coronary artery structure

1 substance findings: iodixanol

EP 4 376 014 A1

FIG 6

$P_1, P_2, ..., P_r$

SB

INT

FCL

LB

NB

ENC

AB

xN

NB

SAB

PE ⊕

EB

NN

INPT

FIG 7

Europäisches Patentamt

European Patent Office

Office européen des brevets

## EUROPEAN SEARCH REPORT

Application Number

**EP 23 15 9107**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/350961 A1 (CSURKA GABRIELA [FR] ET AL) 27 November 2014 (2014-11-27)<br>* abstract; figure 5 *<br>* paragraph [0010] – paragraph [0013] *<br>* paragraph [0022] *<br>* paragraph [0067] – paragraph [0135] *<br>_____ | 1-15 | INV.<br>G16H10/60<br>G06F16/20<br>G06F17/00 |
| | | | TECHNICAL FIELDS SEARCHED (IPC)<br><br>G16H<br>G06F |

The present search report has been drawn up for all claims

| Place of search | Date of completion of the search | Examiner |
|---|---|---|
| Munich | 10 August 2023 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                               
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 9107

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2023

| Patent document cited in search report | Publication date | Patent family member(s) | Publication date |
|---|---|---|---|
| US 2014350961 A1 | 27-11-2014 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MIKOLOV et al.** Efficient Estimation of Word Representations in Vector Space. *arXiv:1301.3781v3,* 07 September 2013

- **VASWANI et al.** Attention Is All You Need. *arXiv: 1706.03762,* 12 June 2017